# EUROPEAN PATENT APPLICATION

(11) **EP 1 201 131 A1**
(43) Date of publication of application: **02.05.2002**
(21) Application number: 00123012.7
(22) Date of filing: 23.10.2000
(51) Int. Cl.: A23C 9/12, C12N 11/16

(54) **Method for preparing fermented food products**

(71) Applicant: Société des Produits Nestlé S.A., 1800 Vevey (CH)
(72) Inventor: Bauche, Anne, 1010 Lausanne (CH); de Maleprade, Dominique, 1807 Blonay (CH); Duboc, Philippe, 1006 Lausanne (CH); Neubauer, Heike, 1010 Lausanne (CH); Zink, Ralf, 1801 Le Mont Pelerin (CH)
(74) Representative: Becker Kurig Straus

(57) **Abstract**

The present invention relates to a novel method for preparing fermented food products, wherein use is made of microorganisms having a predetermined amount of dextransucrase bound to the cell surface thereof. In particular, the present invention pertains to the use of cell bound dextransucrase for the manufacturing of fermented food products and to a bacterial concentrate, having dextransucrase bound to its surface.

SEQUENCE LISTING

<110> Societé des Produits Nestlé

<120> Dextransucrase

<130> 80243

<140>
<141>

<160> 2

<170> PatentIn Ver. 2.1

<210> 1
<211> 5454
<212> DNA
<213> Leuconostoc mesenteroides

<400> 1
gcatcggcat tggaagccgt tatactagcg atgttgctta taatttctaa agtatacaaa 60
aagattagaa tatgatgtaa taaataacaa taatatttat ttttaattac tttatattta 120
ttattaccaa cacgatttat ttataaaaat actaatttca ttatttcgta tatttattac 180
gagacatact agtcttgtta aatacctttt ttatatatta tgtgatttta ttttattttt 240
ttattatttt ataatcaata aaattaatga ctaattaata ataattaaca ttgttcactt 300
atcactagat aagttaacaa tgtttttttt tataaaaaaa ttaatgttta taatattaat 360
ataattctct gattgactgt ttttacaatg ttttttaaaa atatgtcgat tgtgtattga 420
aatttttact gtttttacaa ataaattaag ttataatttt tcaatgtaat agaagagaat 480
attataaggg gaaaatttat gccatttaca gaaaaagtaa tgcggaaaaa gctttataaa 540
gttgggaaaa gttgggtagt tggtggggtt tgtgcttttg cactaaccgc ctcatttgct 600
ttagcaacac caagtgtttt gggagacagt agtgtacctg atgtgagtgc gaataacgtt 660
caatctgctt cagataatac aacggatacg cagcagaaca ctacgattac cgaagaaaat 720
gataaagtac agtctgcagc tactaatgac aatgtaacaa cagctgcaag cgacacaacg 780
caatctgctg ataataatgt gacagaaaaa cagtcagatg atcatgcact tgataatgaa 840
aaagtcgata acaaacaaga tgaagtcgct caaaccaatg ttactagcaa aaatgaggaa 900
tcagcagttg cttcaactga cactgatcct gctgaaacga caactgacga aacacaacaa 960
gttagcggca agtacgttga gaaagacggt agttggtatt attattttga tgatggaaaa 1020
aatgctaaag gtttatcaac gatagacaac aatattcaat attttgacga gagtggtaaa 1080
caagtcaaag gacagtatgt cacaattgat aatcaaacat attattttga taaggactca 1140
ggtgatgagt taactggtct gcaaagcatt gatgggaaca tagttgcttt taacgatgaa 1200
gggcaacaaa tttttaatca atattaccaa tctgaaaacg gtacaacata ctactttgat 1260
gataaaggac acgctgctac cggtattaag aatatcgagg gcaaaaatta ttattttgat 1320
aatcttgggc aactaaaaaa aggcttctct ggtgtgattg atggtcaaat aatgacattt 1380
gatcaggaaa cagggcaaga agtttctaac acaacttctg aaataaaaga aggtttgacg 1440
acacaaaaca cggattatag cgaacataat gcagcccacg gtacggatgc tgaggacttt 1500
gaaaatattg acggctattt aacagctagt tcatggtatc gtccaacaga tattttacgt 1560
aacggaacag actgggaacc ttctacagat acagatttca gaccaatatt gtcagtgtgg 1620

tggccagata agaacaccca ggtcaattat ttaaattaca tggctgattt agggtttatc 1680
agtaatgcgg acagttttga aactggggat agccaaagct tattaaatga agcaagtaac 1740
tatgttcaaa aatcaattga aatgaaaatt agtgcgcaac aaagtacaga gtggttaaag 1800
gatgcgatgg cggctttcat tgtcacgcaa ccacagtgga atgagactag tgaagatatg 1860
agcaatgacc atttacaaaa tggcgcatta acttatgtca acagtccact gacacctgac 1920
gctaattcaa actttagact acttaatcgg acaccaacaa accagactgg tgaacaagcg 1980
tataatttag ataattcaaa aggtggtttt gaattgttgt tagccaatga cgttgataat 2040
tcaaaccctg tagtacaagc agaacaattg aattggttat attatttaat gaattttggt 2100
acgattacgg ccaacgacgc ggatgctaat tttgatggta ttcgtgtaga tgcagtcgac 2160
aatgtggatg ctgatttgtt acaaattgct gccgattatt tcaaactagc ttacggtgtt 2220
gatcaaaatg attctactgc taatcagcat ctttcaattt tggaagattg gagtcacaat 2280
gatcctttgt atgtaacaga tcaaggcagt gaccaattaa ccatggacga ttatgtgcac 2340
acacaattaa tctggtctct aacaaaatca tctgacatac gaggtacaat gcagcgcttc 2400
gtggattatt atatggttga tcgatctaat gatagtacag aaaacgaagc aattcctaat 2460
tacagctttg tacgtgcaca cgacagtgaa gtgcaaacgg ttattgccca aattgtttct 2520
gatttgtatc ctgatgttga aaatagtttg gcaccaacaa cggaacaatt ggcagctgct 2580
ttcaaagtat acaatgaaga tgaaaaatta gcagacaaaa agtacacaca atataatatg 2640
gctagtgctt atgcgatgtt gctaaccaac aaggatactg ttcctcgtgt ctattatggc 2700
gatttatata cagatgatgg tcaatatatg gcaacaaagt caccatacta tgatgcgatt 2760
aacactttgc tgaaggctag agtccaatat gttgctggtg gccaatcgat gtccgttgat 2820
agtaatgacg tgttaacaag tgttcgctat ggtaaagatg ccatgacggc ttctgacact 2880
ggaacatctg agacgcgtac tgaaggtgtt ggggtcatcg tcagcaacaa cgcggagcta 2940
caattagagg atgggcatac agtcacattg cacatggggg cagctcataa gaaccaagct 3000
tatcgtgctt tgttatcaac aactgcagat ggattagctt attatgatac tgatgaaaat 3060
gcacctgtgg cgtacacaga tgctaacggc gatttgattt ttacgaatga atcaatttat 3120
ggtgtacaaa atccacaagt ttctggttac ttggcagttt gggttccggt aggtgcgcaa 3180
caagatcaag atgcacgaac ggcctctgat acaacaacaa acacgagtga taaagtgttc 3240
cattcaaacg ctgctcttga ttctcaagtc atctacgaag gtttctcaaa cttccaagca 3300
tttgctacag acagcagtga atatacaaac gtagtcatcg ctcagaatgc ggaccaattt 3360
aagcaatggg gtgtgacaag cttccaattg gcaccacaat atcgttcaag tacagataca 3420
agtttcttgg attcaattat tcaaaacggg tatgcattca cggatcgtta tgacttaggt 3480
tatggcacac cgacaaaata tggaactgct gatcagttgc gcgatgctat taaagcctta 3540
catgctagcg gtattcaagc cattgccgat tgggtgccgg accaaattta taatttgcca 3600
gagcaagaat tagctactgt cacaagaaca aattcatttg gagatgacga tacagattct 3660
gatattgaca atgccttata tgttgtacaa agtcgtgggg gtggtcaata ccaagagatg 3720
tatggtggtg ccttcttaga agagttacag gcactctatc catccctatt taaagtgaat 3780
caaatctcaa ctggcgttcc aattgatggc agtgtaaaga ttactgagtg ggcggctaag 3840
tacttcaatg gctctaatat ccaaggtaaa ggtgcgggat acgtattgaa agatatgggt 3900
tctaataagt actttaaggt cgtttcgaac actgaggatg gtgactactt accaaaacag 3960
ttaactaatg atctgtcaga aactggcttt acacacgatg ataaaggaat catctattat 4020
acattaagtg gttatcgtgc ccaaaatgca tttattcaag atgatgataa taactattac 4080
tattttgata aaacaggtca tttagtaaca ggtttgcaaa agattaataa ccatacctac 4140
ttcttcttac ctaatggtat cgaactggtc aagagcttct tacaaaacga agatggtaca 4200
attgtttatt tcgataagaa aggtcatcaa gtttttgacc aatatataac tgatcaaaat 4260
ggaaatgcgt attactttga tgatgctggt gtaatgctta aatcagggct tgcaacgatt 4320
gatggacatc aacagtattt tgatcaaaat ggtgtgcagg ttaaggataa gtttgtgatt 4380
ggcactgatg gttataagta ttactttgaa ccaggttgtg gtaacttagc tatcctacgt 4440
tacgtgcaaa acagtaagaa tcaatggttc tattttgatg gtaatggcca tgctgtcact 4500

ggtttccaaa caattaatgg taaaaaacaa tatttctata atgatggtca tcaaagtaaa 4560
ggtgaattca ttaatgcaga cggtgatact ttctatacga gtgccactga tggtcgccta 4620
gtaactggtg ttcagaagat taatggtatt acctatgcgt ttgataacac aggaaatttg 4680
atcacaaatc agtattatca attagcagat ggtaaatata tgttgttaga tgatagtggc 4740
cgtgcgaaaa cagggtttgt attgcaagat ggtgtactaa ggtacttcga tcaaaacggt 4800
gagcaagtga aagatgctat cattgtggat ccagatacta acttgagtta ttatttcaat 4860
gcaacacaag gtgtcgctgt aaaaaatgat tatttcgagt atcaaggtaa ttggtattta 4920
acagatgcta attatcaact tatcaaaggt tttaaagcag ttgacgacag cttacaacat 4980
tttgatgaag tcactggtgt acaaacaaaa gaaagtgctt taataagtgc tcagggtaag 5040
gtttaccaat ttgataataa tggaaatgct gtgtcagcat aagctttctg tatatatagt 5100
gaaaagccaa aggtcctttt gacctttggc tttttgattt aattgtctat ttgaatttcc 5160
ttgatgatat cggctaattt aattttattc atttcttttt cggcagcttc ttgtacttga 5220
ttadagtatt tatccaaagt tgtctgaatg tsactaccaa cattacaatt ttgatttgtt 5280
ttggtgtcaa ttttgagaaa tgaggtgtct tcctccacag cgcgataaat atctaataaa 5340
ctaattgtgt cggctggacg agctaatttt ggatctgcac gtccattact agataataaa 5400
caaccatcat ttttgagctg tgacataatc cgtcgaataa ccacggggtt agag 5454

<210> 2
<211> 1527
<212> PRT
<213> Leuconostoc mesenteroides

<400> 2
Met Pro Phe Thr Glu Lys Val Met Arg Lys Lys Leu Tyr Lys Val Gly
1               5                   10                  15

Lys Ser Trp Val Val Gly Gly Val Cys Ala Phe Ala Leu Thr Ala Ser
            20                  25                  30

Phe Ala Leu Ala Thr Pro Ser Val Leu Gly Asp Ser Ser Val Pro Asp
        35                  40                  45

Val Ser Ala Asn Asn Val Gln Ser Ala Ser Asp Asn Thr Thr Asp Thr
    50                  55                  60

Gln Gln Asn Thr Thr Ile Thr Glu Glu Asn Asp Lys Val Gln Ser Ala
65                  70                  75                  80

Ala Thr Asn Asp Asn Val Thr Thr Ala Ala Ser Asp Thr Thr Gln Ser
                85                  90                  95

Ala Asp Asn Asn Val Thr Glu Lys Gln Ser Asp Asp His Ala Leu Asp
            100                 105                 110

Asn Glu Lys Val Asp Asn Lys Gln Asp Glu Val Ala Gln Thr Asn Val
        115                 120                 125

Thr Ser Lys Asn Glu Glu Ser Ala Val Ala Ser Thr Asp Thr Asp Pro
    130                 135                 140

Ala Glu Thr Thr Thr Asp Glu Thr Gln Gln Val Ser Gly Lys Tyr Val
145                 150                 155                 160

Glu Lys Asp Gly Ser Trp Tyr Tyr Tyr Phe Asp Asp Gly Lys Asn Ala
                165                 170                 175

Lys Gly Leu Ser Thr Ile Asp Asn Asn Ile Gln Tyr Phe Asp Glu Ser
            180                 185                 190

Gly Lys Gln Val Lys Gly Gln Tyr Val Thr Ile Asp Asn Gln Thr Tyr
        195                 200                 205

Tyr Phe Asp Lys Asp Ser Gly Asp Glu Leu Thr Gly Leu Gln Ser Ile
    210                 215                 220

Asp Gly Asn Ile Val Ala Phe Asn Asp Glu Gly Gln Gln Ile Phe Asn
225                 230                 235                 240

Gln Tyr Tyr Gln Ser Glu Asn Gly Thr Thr Tyr Tyr Phe Asp Asp Lys
                245                 250                 255

Gly His Ala Ala Thr Gly Ile Lys Asn Ile Glu Gly Lys Asn Tyr Tyr
            260                 265                 270

Phe Asp Asn Leu Gly Gln Leu Lys Lys Gly Phe Ser Gly Val Ile Asp
        275                 280                 285

Gly Gln Ile Met Thr Phe Asp Gln Glu Thr Gly Gln Glu Val Ser Asn
    290                 295                 300

Thr Thr Ser Glu Ile Lys Glu Gly Leu Thr Thr Gln Asn Thr Asp Tyr
305                 310                 315                 320

Ser Glu His Asn Ala Ala His Gly Thr Asp Ala Glu Asp Phe Glu Asn
                325                 330                 335

Ile Asp Gly Tyr Leu Thr Ala Ser Ser Trp Tyr Arg Pro Thr Asp Ile
            340                 345                 350

Leu Arg Asn Gly Thr Asp Trp Glu Pro Ser Thr Asp Thr Asp Phe Arg
        355                 360                 365

Pro Ile Leu Ser Val Trp Trp Pro Asp Lys Asn Thr Gln Val Asn Tyr
    370                 375                 380

Leu Asn Tyr Met Ala Asp Leu Gly Phe Ile Ser Asn Ala Asp Ser Phe
385                 390                 395                 400

Glu Thr Gly Asp Ser Gln Ser Leu Leu Asn Glu Ala Ser Asn Tyr Val
                405                 410                 415

Gln Lys Ser Ile Glu Met Lys Ile Ser Ala Gln Gln Ser Thr Glu Trp
            420                 425                 430

Leu Lys Asp Ala Met Ala Ala Phe Ile Val Thr Gln Pro Gln Trp Asn
        435                 440                 445

Glu Thr Ser Glu Asp Met Ser Asn Asp His Leu Gln Asn Gly Ala Leu
    450                 455                 460

Thr Tyr Val Asn Ser Pro Leu Thr Pro Asp Ala Asn Ser Asn Phe Arg
465                 470                 475                 480

Leu Leu Asn Arg Thr Pro Thr Asn Gln Thr Gly Glu Gln Ala Tyr Asn
                485                 490                 495

Leu Asp Asn Ser Lys Gly Gly Phe Glu Leu Leu Leu Ala Asn Asp Val
            500                 505                 510

Asp Asn Ser Asn Pro Val Val Gln Ala Glu Gln Leu Asn Trp Leu Tyr
        515                 520                 525

Tyr Leu Met Asn Phe Gly Thr Ile Thr Ala Asn Asp Ala Asp Ala Asn
    530                 535                 540

Phe Asp Gly Ile Arg Val Asp Ala Val Asp Asn Val Asp Ala Asp Leu
545                 550                 555                 560

Leu Gln Ile Ala Ala Asp Tyr Phe Lys Leu Ala Tyr Gly Val Asp Gln
                565                 570                 575

Asn Asp Ser Thr Ala Asn Gln His Leu Ser Ile Leu Glu Asp Trp Ser
            580                 585                 590

His Asn Asp Pro Leu Tyr Val Thr Asp Gln Gly Ser Asp Gln Leu Thr
        595                 600                 605

Met Asp Asp Tyr Val His Thr Gln Leu Ile Trp Ser Leu Thr Lys Ser
    610                 615                 620

Ser Asp Ile Arg Gly Thr Met Gln Arg Phe Val Asp Tyr Tyr Met Val
625                 630                 635                 640

Asp Arg Ser Asn Asp Ser Thr Glu Asn Glu Ala Ile Pro Asn Tyr Ser
                645                 650                 655

Phe Val Arg Ala His Asp Ser Glu Val Gln Thr Val Ile Ala Gln Ile
            660                 665                 670

Val Ser Asp Leu Tyr Pro Asp Val Glu Asn Ser Leu Ala Pro Thr Thr
        675                 680                 685

Glu Gln Leu Ala Ala Ala Phe Lys Val Tyr Asn Glu Asp Glu Lys Leu
    690                 695                 700

Ala Asp Lys Lys Tyr Thr Gln Tyr Asn Met Ala Ser Ala Tyr Ala Met
705                 710                 715                 720

Leu Leu Thr Asn Lys Asp Thr Val Pro Arg Val Tyr Tyr Gly Asp Leu
                725                 730                 735

Tyr Thr Asp Asp Gly Gln Tyr Met Ala Thr Lys Ser Pro Tyr Tyr Asp
            740                 745                 750

Ala Ile Asn Thr Leu Leu Lys Ala Arg Val Gln Tyr Val Ala Gly Gly
        755                 760                 765

Gln Ser Met Ser Val Asp Ser Asn Asp Val Leu Thr Ser Val Arg Tyr
    770                 775                 780

Gly Lys Asp Ala Met Thr Ala Ser Asp Thr Gly Thr Ser Glu Thr Arg
785                 790                 795                 800

Thr Glu Gly Val Gly Val Ile Val Ser Asn Asn Ala Glu Leu Gln Leu
                805                 810                 815

Glu Asp Gly His Thr Val Thr Leu His Met Gly Ala Ala His Lys Asn
            820                 825                 830

Gln Ala Tyr Arg Ala Leu Leu Ser Thr Thr Ala Asp Gly Leu Ala Tyr
        835                 840                 845

Tyr Asp Thr Asp Glu Asn Ala Pro Val Ala Tyr Thr Asp Ala Asn Gly
    850                 855                 860

Asp Leu Ile Phe Thr Asn Glu Ser Ile Tyr Gly Val Gln Asn Pro Gln
865                 870                 875                 880

Val Ser Gly Tyr Leu Ala Val Trp Val Pro Val Gly Ala Gln Gln Asp
                885                 890                 895

Gln Asp Ala Arg Thr Ala Ser Asp Thr Thr Thr Asn Thr Ser Asp Lys
            900                 905                 910

Val Phe His Ser Asn Ala Ala Leu Asp Ser Gln Val Ile Tyr Glu Gly
        915                 920                 925

Phe Ser Asn Phe Gln Ala Phe Ala Thr Asp Ser Ser Glu Tyr Thr Asn
    930                 935                 940

Val Val Ile Ala Gln Asn Ala Asp Gln Phe Lys Gln Trp Gly Val Thr
945                 950                 955                 960

Ser Phe Gln Leu Ala Pro Gln Tyr Arg Ser Ser Thr Asp Thr Ser Phe
                965                 970                 975

Leu Asp Ser Ile Ile Gln Asn Gly Tyr Ala Phe Thr Asp Arg Tyr Asp
            980                 985                 990

Leu Gly Tyr Gly Thr Pro Thr Lys Tyr Gly Thr Ala Asp Gln Leu Arg
        995                 1000                1005

Asp Ala Ile Lys Ala Leu His Ala Ser Gly Ile Gln Ala Ile Ala Asp
    1010                1015                1020

Trp Val Pro Asp Gln Ile Tyr Asn Leu Pro Glu Gln Glu Leu Ala Thr
1025                1030                1035                1040

Val Thr Arg Thr Asn Ser Phe Gly Asp Asp Asp Thr Asp Ser Asp Ile
                1045                1050                1055

Asp Asn Ala Leu Tyr Val Val Gln Ser Arg Gly Gly Gly Gln Tyr Gln
            1060                1065                1070

Glu Met Tyr Gly Gly Ala Phe Leu Glu Glu Leu Gln Ala Leu Tyr Pro
        1075                1080                1085

Ser Leu Phe Lys Val Asn Gln Ile Ser Thr Gly Val Pro Ile Asp Gly
    1090                1095                1100

Ser Val Lys Ile Thr Glu Trp Ala Ala Lys Tyr Phe Asn Gly Ser Asn
1105                1110                1115                1120

Ile Gln Gly Lys Gly Ala Gly Tyr Val Leu Lys Asp Met Gly Ser Asn
                1125                1130                1135

Lys Tyr Phe Lys Val Val Ser Asn Thr Glu Asp Gly Asp Tyr Leu Pro
            1140                1145                1150

Lys Gln Leu Thr Asn Asp Leu Ser Glu Thr Gly Phe Thr His Asp Asp
        1155                1160                1165

Lys Gly Ile Ile Tyr Tyr Thr Leu Ser Gly Tyr Arg Ala Gln Asn Ala
    1170                1175                1180

Phe Ile Gln Asp Asp Asp Asn Asn Tyr Tyr Tyr Phe Asp Lys Thr Gly
1185                1190                1195                1200

His Leu Val Thr Gly Leu Gln Lys Ile Asn Asn His Thr Tyr Phe Phe
                1205                1210                1215

Leu Pro Asn Gly Ile Glu Leu Val Lys Ser Phe Leu Gln Asn Glu Asp
            1220                1225                1230

Gly Thr Ile Val Tyr Phe Asp Lys Lys Gly His Gln Val Phe Asp Gln
        1235                1240                1245

Tyr Ile Thr Asp Gln Asn Gly Asn Ala Tyr Tyr Phe Asp Asp Ala Gly
    1250                1255                1260

Val Met Leu Lys Ser Gly Leu Ala Thr Ile Asp Gly His Gln Gln Tyr
1265                1270                1275                1280

Phe Asp Gln Asn Gly Val Gln Val Lys Asp Lys Phe Val Ile Gly Thr
                1285                1290                1295

Asp Gly Tyr Lys Tyr Tyr Phe Glu Pro Gly Cys Gly Asn Leu Ala Ile
            1300                1305                1310

Leu Arg Tyr Val Gln Asn Ser Lys Asn Gln Trp Phe Tyr Phe Asp Gly
        1315                1320                1325

Asn Gly His Ala Val Thr Gly Phe Gln Thr Ile Asn Gly Lys Lys Gln
    1330                1335                1340

Tyr Phe Tyr Asn Asp Gly His Gln Ser Lys Gly Glu Phe Ile Asn Ala
1345                1350                1355                1360

Asp Gly Asp Thr Phe Tyr Thr Ser Ala Thr Asp Gly Arg Leu Val Thr
                1365                1370                1375

Gly Val Gln Lys Ile Asn Gly Ile Thr Tyr Ala Phe Asp Asn Thr Gly
            1380                1385                1390

Asn Leu Ile Thr Asn Gln Tyr Tyr Gln Leu Ala Asp Gly Lys Tyr Met
        1395                1400                1405

Leu Leu Asp Asp Ser Gly Arg Ala Lys Thr Gly Phe Val Leu Gln Asp
    1410                1415                1420

Gly Val Leu Arg Tyr Phe Asp Gln Asn Gly Glu Gln Val Lys Asp Ala
1425                1430                1435                1440

Ile Ile Val Asp Pro Asp Thr Asn Leu Ser Tyr Tyr Phe Asn Ala Thr
                1445                1450                1455

Gln Gly Val Ala Val Lys Asn Asp Tyr Phe Glu Tyr Gln Gly Asn Trp
            1460                1465                1470

Tyr Leu Thr Asp Ala Asn Tyr Gln Leu Ile Lys Gly Phe Lys Ala Val
        1475                1480                1485

Asp Asp Ser Leu Gln His Phe Asp Glu Val Thr Gly Val Gln Thr Lys
    1490                1495                1500

Glu Ser Ala Leu Ile Ser Ala Gln Gly Lys Val Tyr Gln Phe Asp Asn
1505                1510                1515                1520

Asn Gly Asn Ala Val Ser Ala
                1525

## Description

The present invention relates to a novel method for preparing fermented milk products, wherein use is made of microorganisms having a predetermined amount of dextransucrase bound to the cell surface. In particular, the present invention pertains to the use of cell bound dextransucrase for the manufacturing of fermented milk products and to bacterial concentrates containing microorganisms having dextransucrase bound to their surface.

In the preparation of food products, in particular dairy products, use is made of various micro-organisms, the fermentative activity thereof is relied upon (for a general review see Encyclopedia of Food Microbiology, R.K. Robinson Editor, Academic Press, 2000). As an example, in the preparation of a yogurt or fermented milk products in general, milk, that has been pasteurized and that may have been supplemented with various milk proteins, is inoculated with a lactic acid bacterium up to a final concentration of 10⁵-10⁹ colony forming units (cfu) per ml and the liquid is incubated at 39-44°C for about 4-8 hrs. During the fermentation period lactose is mainly converted to lactic acid with the pH of the liquid slowly decreasing. Concurrently, milk proteins are partially degraded and metabolized and the cells multiply up to final concentration of 10⁷-10⁹ cell forming units (cfu) per ml final product. At a pH of about 5.2 casein proteins start to coagulate with the liquid turning into a gel-like consistency/texture. Some strains of lactic acid bacteria may also synthesize exopolysaccharides (EPS) which contribute to the texture development. The fermentation is eventually stopped when a desired pH of about 4.4-4.6 is achieved by rapidly cooling the product obtained to a temperature of about 4°C to stop the metabolic activity of the bacteria contained therein.

A problem involved in the preparation of fermented milk products, such as yogurt, resides in that the texture of the product resulting from the fermentative process alone is often not appealing for the consumer. Therefore, in order to improve the texture of the product thickening agents, such as e.g. modified starch, alginate, carrageenan or locust bean gum are added to the product in order to increase its consistency. Yet, proceeding accordingly is not always possible, since e.g. in case of a yogurt, the addition and distribution of the thickening agent will destroy the gel-like texture already produced during the fermentation.

Another approach to overcome this problem is to use strains of lactic acid bacteria that produce polysaccharides, which in turn may serve as thickeners. A drawback of this approach lies in that there is only a limited number of microorganisms available for use in the preparation of food material that produce such polysaccharides and that fermentation conditions greatly affect polysaccharide production. In addition, since the production of the polysaccharides may not be predicted and is most often not sufficient at all, a reliable good quality of the resulting products cannot be ensured.

Another way to overcome the above problem is to add an enzyme forming the polysaccharides in an isolated form to the medium during the fermentation process. However, also this approach proved to be unsatisfactory, since enzyme purification might be tedious and costly, and it is also difficult to evenly distribute the enzyme in the medium, which is a prerequisite for obtaining an uniform texture of the resulting product. In particular, the enzyme has to be added in a powdered form so as not to introduce additional liquid. However, dissolving the enzyme in the medium poses problems in that the dissolution has to be performed in a manner sufficient and quick to avoid formation of clumps. In case the enzyme is added to the medium at an early stage of the fermentation said clumps of enzymes often do not sufficiently dissolve to avoid formation of regions in the product having a more rigid texture as compared to other regions of the product exhibiting a more soft texture. On the other hand, if the enzyme is added to the medium at a late stage of the fermentative process the already started coagulation of the protein material contained therein will often prevent the enzymatic material to get evenly distributed throughout the medium, again, bringing about a texture of the product with regions of softer and more rigid consistency. Moreover, it is important to control enzyme activity during the process and to inactivate the enzyme at a desired time point in a way compatible with the process without destroying the developed texture.

Accordingly, an objective of the present invention resides in overcoming the above problems of the prior art and providing a novel method for the preparation of fermented food products, which enables an easier manufacturing of fermented food products, having an appealing and uniform texture.

According to the present invention the above problem has been solved by providing a method for manufacturing fermented food products, which comprises the step of prior to the fermentative process, contacting the microorganism to be used for the fermentation process with a predetermined amount of a dextransucrase and under conditions, that the dextransucrase binds to the cell surface thereof. The microorganisms such treated may then be included in a common fermentation process.

During the extensive studies leading to the present invention it has been surprisingly found that dextransucrase may be quickly bound to the cell surface of a variety of gram-negative and gram-positive microorganisms under a broad range of temperature and pH-conditions and that the enzyme will still be active even when brought in contact with the corresponding substrates. Accordingly, a food grade microorganism with the enzyme bound on its cell surface may be directly used for the manufacture of fermented food material, with the microorganism developing its fermentative activity in the medium serving at the same time as a carrier for the enzyme thus facilitating the transfer thereof into the fermentation medium. Since the microorganisms will be well distributed in the medium, this applies likewise to the enzyme they carry with them. Consequently, the products obtained by using such microorganisms exhibit an uniform texture/consistency. In addition, since the amount of dextransucrase may be precisely predetermined, the texture of the fermented food product may be controlled in an efficient manner.

According to a preferred embodiment the amount of dextransucrase bound to the respective micro-organisms ranges from about 0.001 to 0.5 U/10⁶ cfu.

According to yet another preferred embodiment the microorganisms to be used in the preparation of the food material are selected from the group consisting of *Lactobacillus, Bifidobacterium, Streptococcus, Leuconostoc, Pediococcus, Enterococcus* and are more preferably *Lactobacillus johnsonii* (CNCM I-1225, CNCM I-2369 and CNCM I-2474), *Lactobacillus paracasei* (CNCM I-2116), *Lactobacillus acidophilus* (CNCM I-2332), *Lactobacillus reuteri, Lactobacillus* *animalis, Lactobacillus ruminis, Lactobacillus casei, Lactobacillus rhamnosus, Lactobacillus fermentum, Lactobacillus bulgaricus, Lactobacillus plantarum, Bifidobacterium bifidum* (CNCM I-2333), *Bifidobacterium infantis* (CNCM I-2334), *Bifidobacterium adolescentis* (CNCM I-2168), *Bifidobacterium longum, Bifidobacterium lactis* (ATCC 27536), *Bifidobacterium bifidum, Bifidobacterium animalis, Streptococcus thermophilus.* The above specifically mentioned microorganisms have all be deposited according to the Budapest Treaty and are available from the Institute Pasteur, France.

The microorganisms pre-treated in such manner may be used for the preparation of a variety of different dairy products (such as yoghurt, curd, cheese, fermented milks, milk based fermented products, milk based powders, ice cream), non-dairy food products (such as fermented cereal based products, drinks, water-based jellies, fruit juice based jellies), infant formula or pet food. Due to the capacity of the microorganisms to evenly distribute the dextransucrase in the medium to be fermented and the concurrent potential for forming the thickening agent *in-situ,* the resulting product shows an excellent uniform texture.

Another advantage of the invention is not to require coagulation of milk to achieve a satisfactory texture with the consequence that less acidic end-products may be obtained. Likewise, the same approach can be used to give texture to non-dairy fermented products like fruit and vegetable juices etc.

The dextransucrase may be bound to the respective lactic acid bacterium to be used for the fermentation in an easy manner by simply contacting a predetermined amount of the enzyme with a given number of viable cells in a medium suitable for the enzyme to bind to the cell surface of the microorganism. It has been surprisingly recognized that dextransucrase quickly binds to the cell surface of a variety of different microorganisms, even under a broad range of different pH-and temperature conditions. Homogeneous mixing of the enzyme with the microorganisms will ensure an even distribution, so that the microorganisms will essentially carry the same amount of enzyme bound on their surface.

The bacteria thus obtained may then be stored until further use or concentrated in a suitable manner. According to yet another preferred embodiment, the present invention provides a bacterial concentrate in liquid or in powdered form, or a bacterial solution, which concentrate/solution may be simply added to a medium to be fermented so that upon inoculating the medium with the respective micro-organism a concurrent inoculation with dextransucrase takes place.

According to a broad aspect of the invention the part of the dextransucrase enzyme, that binds to the surface of the microorganisms may be used to include biologically active substances in a food material. In its easiest form the biologically active substance is the enzymatic part of the dextransucrase protein itself. Yet, also other substances, such as health promoting agents, e.g. vitamins, oral vaccines etc. may be linked to the binding region of dextransucrase and added to food products by using the microorganisms as a carrier.

To this end, a compound of interest is linked with the dextransucrase itself or a part thereof, binding to the bacterial cell wall and the combined molecule is then bound to the bacteria. Methods and techniques for combining proteinaceous materials with other compounds are well known to the skilled person. Based upon his own technical skill he will select the appropriate method. In case an additional proteinaceous material shall be linked to dextransucrase or a part thereof binding to the bacterial cell wall, the corresponding DNA region(s) coding for the respective polypeptides may be combined in a vector and may be expressed in a suitable manner.

In effect, the binding part of the dextransucrase enzyme will eventually serve for enabling the binding of the enzymatic activity of the dextransucrase itself to the microorganism and/or for enabling the binding of another activity of some biologically active compound of interest.

In the figures,
Fig. 1 shows the results obtained by binding dextransucrase to different types of cells;
Fig. 2 shows the kinetics of dextransucrase binding onto *L*. *johnsonii* La1;
Fig.3 shows the stability of the binding of dextransucrase onto *L*. *johnsonii* La1 at different temperatures;
Fig. 4 shows a removal of dextransucrase bound to cells of *L*. *johnsonii* La1 by washing with binding buffer;
Fig. 5 shows the influence of the pH on binding capacity of dextransucrase onto *L*. *johnsonii* La1;
Fig. 6 shows the influence of the incubation temperature on binding of dextransucrase onto *L. johnsonii* La1;
Fig. 7 shows the binding capacity *of L. johnsonii* La1 cells during cell growth;
Fig. 8 shows the influence of dextran formation on cell binding of DsrD;
Fig. 9 shows the binding stability of dextransucrase onto *L*. *johnsonii* La1 in the presence of sucrose;
Fig. 10 shows the ratio of dextransucrase activity bound to *L*. *johnsonii* La1 cells or free in the supernatant;
Fig. 11 shows the ratio of dextransucrase activity bound to *L*. *mesenteroides* Lcc4 cells or free in the supernatant;
Fig. 12 shows the results obtained when binding dextransucrase to peptidoglycan in a microtiter plate;
Fig. 13 shows the flow rate through nozzle to estimate viscosity of fermented milk;
Fig. 14 shows the results of a sensory evaluation of samples A-F prepared according to the invention, wherein Bars indicate standard deviation of the panel of 7 people.; and

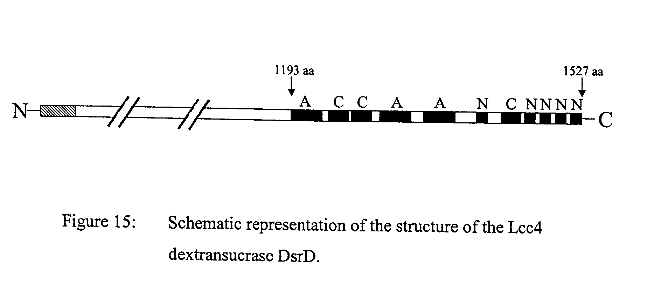

Fig. 15 schematically shows the structure of the DsrS gene *of L. mesenteroides* NRRL-B512-F.

In the experiments leading to the invention the present inventors have tried to characterize the dextransucrase gene *dsrD* of *L. mesenteroides* Lcc4, which strain is available from the Institute Pasteur (accession no. CNCM I-1292).

So far, three dextransucrases of two different *Leuconostoc* strains have been published (WO 89/12386; Monchois et al., Gene 182:23-32, 1996; Monchois et al., FEMS Microbiol. Lett. 159:307-315, 1996; Monchois et al., Appl. Env. Microbiol. 64 (1998), 1644-1649). It has been shown that dextransucrases are closely related and have a common structure. They are large enzymes with an average molecular weight of 160.000 Dalton and are composed of two different functional domains. The N-terminal catalytic domain (about 900 aa) is responsible for the cleavage of sucrose whereas the C-terminal domain (300-400 aa) composed of a series of homologous directly repeating units is involved in glucan binding.

The respective gene *dsrD* of *L. mesenteroides* Lcc4 strain has been isolated from a DNA library prepared according to the techniques illustrated in Maniatis et al. (A Laboratory Manual, Cold Spring Harbor, 1992) using degenerate PCR primers. An alignment of the three *L*. *mesenteroides* dextransucrase genes *dsrS* (WO 89/12386), *dsrA* (Monchois et al., 1996, Gene 182:23-32), and *dsrB* (Monchois et al., 1998, FEMS Microbiol. Lett. 159:307-315) identified several conserved regions. Three of the conserved regions were used to design two sets of degenerate primers for specific amplification of internal portions of internal portions of the dextransucrase gene of *L. mesenteroides* Lcc4.

A sequence analysis of the present gene revealed that the dextransucrase gene of Lcc4 (4.6 kb, SEQ ID No. 1) codes for a protein of 1527 aa (SEQ ID No. 2). The amino acid sequence is highly similar to the other known dextransucrases DsrS (WO 89/12386), DsrA and DsrB (Monchois et al., Appl. Env. Microbiol. 64 (1998), 1644-1649) and reveals an organization typical for dextransucrases, i.e. two functional domains, the N-terminal portion extending from amino acid 268 to 1134, which resembles the corresponding region of glucosyltransferases (GTFs) and is necessary for dextransucrase activity. This region includes a putative catalytic site for sucrose hydrolysis. The C-terminal portion of GTFs which contains about 300 to 400 amino acids does not participate in sucrose splitting but is responsible for glucan-binding. The glucan-binding region is composed of a series of homologous repeating units each consisting of 20 to 30 amino acids. By virtue of sequence similarity, the repeated sequences have been classified into four different classes (A-D). In the sequence of the dextransucrase DsrS of *L. mesenteroides* B-512F only repeats of the classes A and C have been identified next to a series of small repeats which have been named N-repeats. In contrast thereto, all of the repeats contain the same structural element, designated the 'YG repeat'. It is characterized by the presence of clusters of aromatic residues, the prevalence of polar and turn-promoting residues at certain positions, and the common occurrence of a glycine residue located three or four residues downstream from the aromatic cluster. A consensus motif has been presented as NDGYYFxxxGxxH⁰x(G/N)x H⁰H⁰H⁰ in which x are non-conserved amino acid residues and H⁰ hydrophobic amino acid resides.

The presence of a signal peptide indicated that the dextransucrase of Lcc4 is a secretory protein. In agreement with this, the Lcc4 dextransucrase protein was identified in the culture supernatant after fermentation of Lcc4 in the presence of sucrose. SDS-PAGE followed by incubation in a buffered sucrose solution and an activity staining procedure identified a protein with an estimated size of 170 kDa with dextransucrase activity. An N-terminal amino acid analysis finally confirmed that the protein is the identified and sequenced dextransucrase DsrD.

In the following this enzyme was used to carry out the experiment illustrated in the following examples, which are not to be construed to be limiting but to serve for illustrative purposes only.

### Examples

The following materials and Methods have been used:

### Bacterial strains:

*Leuconostoc mesenteroides* Lcc4 (CNCM I-1292), Lcm 15 (NCC 1687), cc53, cc58, *Lactobacillus johnsonii* La1 (CNCM I-1225), *Lactobacillus acidophilus* (ATCC 4356), *Lactobacillus gasseri* (DSM 20243), *Staphylococcus carnosus* TM300, *Bacillus subtilis* (DSM 402), *Serratia plymutica* (DSM 4540), *Aeromonas hydrophila* (ATCC 7966). *Escherichia coli* DH5α was used for the binding studies.

### Media and growth conditions:

### L. mesenteroides Lcc4

This strain was routinely grown at 30°C in MRS (Difco, USA) and for fermentation purposes in Lcc4 medium. The pH was adjusted to 6.7 and the medium was sterilized at 121°C for 15 min.

**Table 1:**

| | | |
|---|---|---|
| Lcc4-suc and Lcc4-glu media | | |

| | Concentration (g/l) | |
|---|---|---|
| Component | Lcc4-suc | Lcc4-glu |
| Sucrose | 20 | - |
| Glucose | - | 60 |
| K₂HPO₄ | 20 | 20 |
| Bacto-peptone | 5 | 5 |
| Yeast extract | 5 | 5 |
| MnSO₄,H₂O | 0.02 | 0.02 |
| NaCl | 0.01 | 0.01 |
| FeSO₄, 7H₂O | 0.01 | 0.01 |
| MgSO₄, 7H₂O | 0.2 | 0.2 |

The pH was adjusted to 6.7 with *orth*o-phosphoric acid 85% (Merck). The medium was sterilized at 121°C for 15 min.

For the preparation of cryoculture inoculum samples of Lcc4, a lyophilized stock culture of *Leuconostoc mesenteroides* Lcc4 was suspended in 1 ml MRS, transferred to a test tube containing 9 ml MRS and incubated overnight at 30°C without agitation. Then a second 10-ml MRS culture was inoculated with 100 µl of the previous inoculum and cultured for 9 hours. The culture was centrifuged for 10 min at 5000 rpm, the pellet was suspended in 1 ml MRS containing 40% glycerol and aliquots of 10 µl were quickly distributed in sterile Eppendorf tubes. The samples for inoculation were stored at -80°C.

For the inoculation of a fermenter, two precultivation steps were performed. A first preculture was obtained by inoculation of 10 ml MRS with 10 µl cryoculture sample and incubation overnight at 30°C without agitation. A second preculture was inoculated with 1% and incubated for 9 hours at 30°C (200 ml medium was used in Erlenmeyer shake flasks agitated at 100 rpm). The main culture was inoculated with 1% (vol./vol.) of this second preculture.

The batch and pH-controlled fed-batch cultures of Lcc4 were performed at 23°C in a 15-liter MBR bioreactor with a 7-liter working volume. The fermenter was sterilized at 121°C for 30 min. Under standard conditions, the fermentation was carried out at an aeration rate of 0.1 vvm air with an agitation of 150 rpm and at a constant pH of 6.7.

For the batch culture, the pH was maintained constant by addition of 10% NaOH, 10% KOH solution. For the pH-controlled fed-batch mode, the standard feeding strategy consisted of addition at identical rate of a base solution (58.7 g/l NaOH, 58.7 g/l KOH) and a concentrated sucrose solution (600 g/l). A pH controller activated both pumps. The aim of this installation was to carry out a pH-controlled fed-batch fermentation. The two feed stock solutions (water for the base preparation) were autoclaved for 15 minutes at 121°C.

### Lactobacillus johnsonii La1:

*L. johnsonii* La1 was anaerobically grown at 37°C in MRS as described above.

### Staphylococcus carnosus TM300:

*S. carnosus* TM300 was cultivated at 37°C in basic medium (Table 2) with shaking.

**Table 2:**

| Basic medium | |
|---|---|
| Component | g/l |
| Casein hydrolysate peptone | 10 |
| Yeast extract | 5 |
| Glucose monohydrate | 1 |
| NaCl | 5 |
| K₂HPO₄ | 1 |

The pH was adjusted to 6.7 and the medium was sterilized at 121°C for 15 min.

### Escherichia coli DH5α:

*Escherichia coli* DH5α was grown at 37°C in Luria Bertani (LB)-medium (Maniatis supra) with shaking. The pH was adjusted to 7.0 and the medium was sterilized at 121°C for 15 min.

### Bacillus subtilis:

*Bacillus subtilis* was cultivated at 37°C in LB medium while shaking.

### Lactobacillus acidophilus ATCC 4356:

*Lactobacillus acidophilus* was anaerobically cultivated at 37°C in MRS without agitation.

### Lactobacillus gasseri DSM 20243:

*Lactobacillus gasseri* was anaerobically cultivated at 37°C in MRS without agitation.

### Serratia plymutica DSM 4540:

*Serratia plymutica* was cultivated at 30 °C in BHI broth (Difco 0037-17) with agitation.

### Aeromonas hydrophila ATCC 7966:

*Aeromonas hydrophila* was cultivated at 30 °C in BHI broth (Difco) under agitation.

### Analytical methods

The sugars sucrose, glucose, fructose in the culture supernatant were determined by HPLC (HP Series 1100, Aminex HPX 87H column with 5mmol H₂SO₄, flow rate 0.6 ml/min, T=35°C, injection of a 20µl sample). The compounds were detected with a refractometer (HP 1047A).

### Enzymatic methods

A standard unit (U) of dextransucrase activity is defined as the amount of enzyme that catalyzes in presence of sucrose the liberation of 1 µmol of D-fructose during dextran formation per min at 30°C.

### Detection of reducing sugars (Nelson-Somogyi assay)

Dextransucrase activity was determined by measuring the liberation of reducing sugars during dextran formation. 10 µl of the dextransucrase sample was mixed with 3 ml of substrate buffer (20 mmol sodium acetate, pH 5.2, 200 mmol sucrose, 20 mmol CaCl₂, 0.2 g/l sodium azide). 1 ml thereof was quickly transferred into a Pyrex tube (1 cm diameter, height 16 cm) and put to - 20°C (control). 1 ml was incubated for 1 hour at 30°C. A mixture of 25 ml solution A (25 g Na₂CO₃, 25 g KNaC₄H₄O₆·H₂O, 20 g NaHCO₃, 200 g Na₂SO₄ anhydr. and 5 g Na-benzoat per liter) and 1 ml of solution B (15 g CuSO₄·5H₂O, 1 to 2 drops of H₂SO₄ per 100 ml) was prepared. After incubation, the Nelson-Somogyi test was performed, by addition of 1 ml reagent A+B to both, the assay and the control and quickly heating them for 30 min at 100°C to stop the dextransucrase reaction. As standard solution, 0.6 mmol fructose in substrate buffer was used. After heat inactivation, 1 ml of solution C (25 g (NH₄)₆Mo₇O₂₄·4H₂0, 21 ml of H₂SO₄, 3 g of Na₂HAsO₄·7H₂O per 500 ml) and 7 ml water was added. The extinction of the different samples at 520 nm is measured against a blank (substrate buffer).

### Binding of dextransucrase to bacterial cell wall components:

Cells were cultivated overnight as described above. A culture volume corresponding to the chosen OD was centrifuged at 6000 rpm 4°C for 10 min. The supernatant was discarded and cells were resuspended in a mixture of culture supernatant containing dextransucrase and binding buffer (20-mmol sodium acetate, pH 5.2, 20 mmol CaCl₂). As a control, the dextransucrase activity of the pellet was determined after incubation in the binding buffer without addition of dextransucrase. The amount of bacterial cells, quantified by the OD, the enzyme activity and the binding conditions (temperature, incubation time) represented the binding parameters. To determine, whether dextransucrase was attached to the bacterial cells, the suspensions were centrifuged (6000 rpm, 4°C, 10 min) and the pellets were resuspended in 100 µl binding buffer. The dextransucrase activity of the pellet fraction was determined as described above.

The ability of dextransucrase to bind cell wall components was assayed in microtiter plates. Solutions were prepared in coating buffer: 4 mg/ml purified peptidoglycans from *Staphylococcus aureus* (Fluka), 0.1 mg/ml lipoteic acid (LTA) from *S. aureus* (Sigma), 0.07 mg/ml purified LTA from *Lactobacillus johnsonii* La1. 50 µl of solution were tranferred into microtiter wells (Nunc, DK) and incubated overnight at 4°C. The wells were washed with 10 mmol PBS (pH 6.4). The ability of purified peptidoglycans to bind to microtiter plates was confirmed with blue dyed peptidoglycans: a slight blue color remained on the plate. After washing 300 µL of blocking solution (10 mg/ml gelatine in 0.1 M Tris, pH 8.0 with 0.01% azide) was added to wells and incubated for 1 h at 37°C. The wells were washed again and 50 µL of Lcc4 culture supernatant containing dextransucrase obtained during a pH-controlled fed-batch were added. After one hour of incubation at 4°C, the wells were washed and 240 µl sucrose buffer was added. Samples (100 µl) were taken at different times after incubation at 30°C, and reducing sugars were determined as above.

### Example 1

### Binding of dextransucrase to different bacterial cells

A binding assay was developed to detect attachment of dextransucrase to a variety of bacterial species (*Leuconostoc mesenteroides species, Bacillus subtilis, Staphylococcus carnosus,* *Lactobacillus johnsonii, Escherichia coli, Lactobacillus acidophilus, Lactobacillus gasseri, Serratia plymutica, Aeromonas hydrophila*).

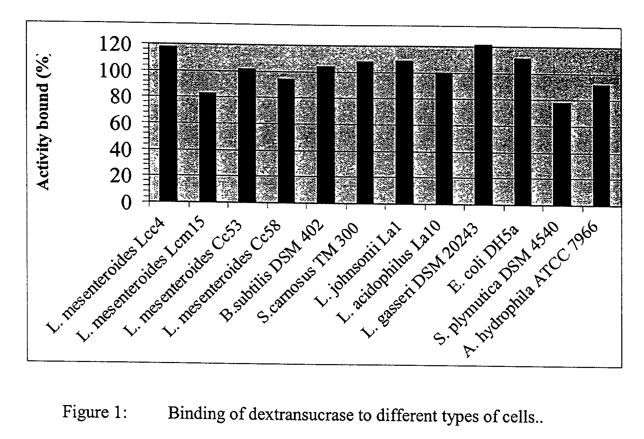

After overnight cultivation, the optical density of the different cultures at 590 nm was determined and an appropriate volume was centrifuged to yield pellets giving an OD of 10 when resuspended in 200 µl of a culture supernatant containing dextransucrase at an enzyme concentration of 1 U/ml. The culture was subsequently incubated on ice for 20 min. As a control, the cells were resuspended in the culture medium containing no dextransucrase. Subsequently, the cells suspensions were centrifuged and the dextransucrase activity of the pellet and the supernatant fractions were determined using the Nelson-Somogyi assay (supra). Table 3 lists the characteristics of the various strains utilized and the results are shown in fig. 1.

**Table 3:**

| Characteristics of the chosen strains | | |
|---|---|---|
| Strains | Gram | S-layer |
| *Leuconostoc mesenteroides* Lcc4, Lcm 15, cc53, cc58, | + | Nd |
| *Lactobacillus johnsonii* La1 | + | no |
| *Lactobacillus acidophilus* ATCC 4356 | + | yes |
| *Lactobacillus gasseri* DSM 20243 | + | no |
| *Staphylococcus carnosus* TM300 | + | no |
| *Bacillus subtilis DSM* 402 | + | Nd |
| *Serratia plymutica DSM* 4540 | - | Nd |
| *Aeromonas hydrophila* ATCC 7966 | - | yes |
| *Escherichia coli* DH5α | - | no |
| Nd = not determined | | |

As becomes evident from Fig. 1, the dextransucrase protein bound efficiently to all bacterial cells tested. After centrifugation, 100% of the activity was found in the cell pellet and no activity was detectable in the supernatant fraction. No dextransucrase activity was observed in the control experiment. *Leuconostoc* strains producing dextransucrase were grown in the absence of the inducer sucrose so as to avoid endogeneous dextransucrase synthesis.

### Example 2

### Kinetics of dextransucrase binding

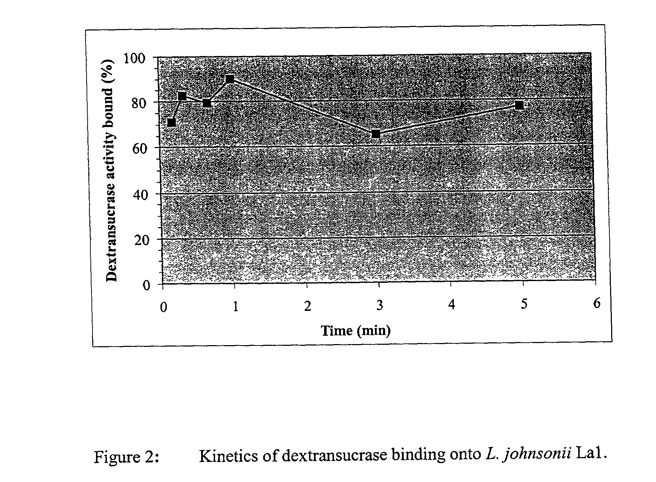

To analyze the binding kinetics, *L*. *johnsonii* La1 cells were incubated up to 5 min with dextransucrase. The binding parameters were an OD of 10, an enzyme concentration of 0.57 U/ml and incubation for up to 10 min at 4°C. After different time intervals, the cell suspension was centrifuged and the cells were washed once with buffer before dextransucrase activity measurement. As may be seen from Fig. 2, already after 10 sec of incubation, the majority of the dextransucrase was attached to the bacterial cells and no further increase could be obtained.

### Example 3

### Stability of the interaction of dextransucrase with the bacterial surface

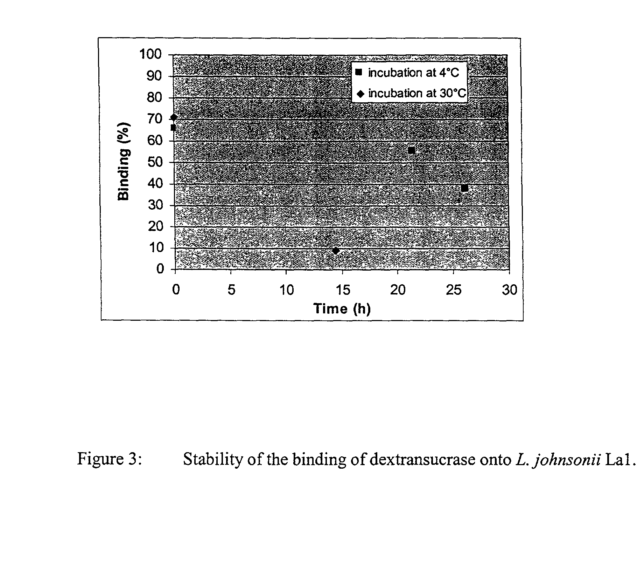

The stability of the interaction was studied for up to 24 hrs at 4°C and for 15 hrs at 30°C. The binding parameters were an OD of 2.0 and an enzyme concentration of 0.3 U/ml. After the incubation periods, the cells were centrifuged to quantify the dextransucrase activity still attached to the cell fraction. As control, a supernatant containing dextransucrase was incubated for the same time period at the respective temperatures. No change in the enzyme activity was detected during incubation at 4°C or 30°C. As may be seen in Fig. 3, the binding was stable for 20 hrs at 4°C. However, at a temperature of 30°C the amount of enzyme attached to the cells decreased significantly, so that after 15 hrs only 10% of the enzyme was still in cell association.

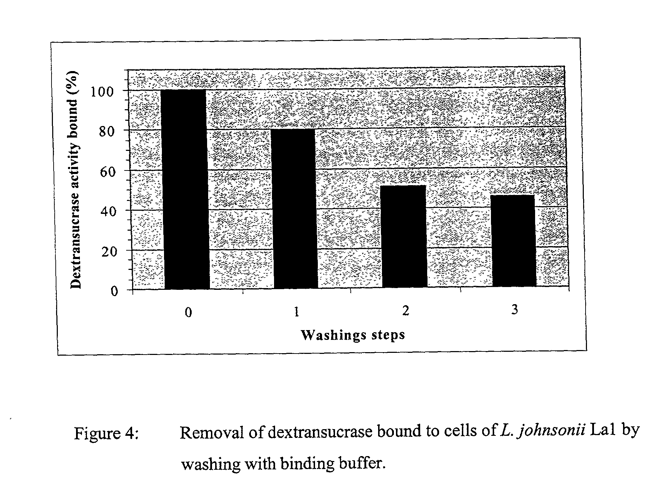

To analyze the stability of the dextransucrase interaction with the bacterial cell surface, it was further tested whether the enzyme could be removed from the surface by subsequent washing with binding buffer. Up to three washing steps with binding buffer were performed. Dextransucrase activity of *Lactobacillus johnsonii* cell suspension was measured directly after binding (The binding parameters : OD of 10, enzyme concentration of 0.57 U/ml and incubation for 10 min at 4°C ) and after each of in total three washing steps. As shown in Fig. 4, the attached enzyme could be gradually removed by washing indicating that dextransucrase is not tightly bound but only non-covalently attached to the bacterial surface. After the third washing step around 50% of enzyme was still in cell association.

### Example 4

### Influence of the pH on the binding of dextransucrase to L. johnsonii La1

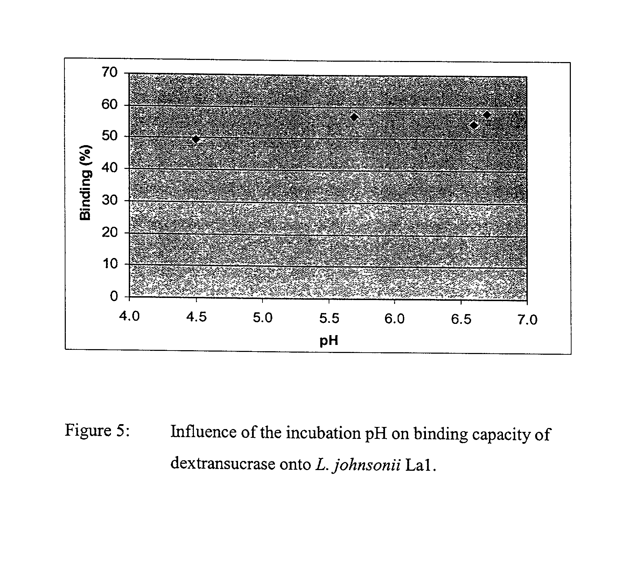

To investigate the influence of the pH on the binding of dextransucrase to *L. johnsonii* La1, the binding reaction was performed at varying pH (4.0, 5.2, 6.7 and 8.0). As depicted in Fig. 5, the binding efficiency remained constant when the pH varied from 4.5 to 6.7.

### Example 5

### Influence of the temperature on the binding of dextransucrase to L. johnsonii La1

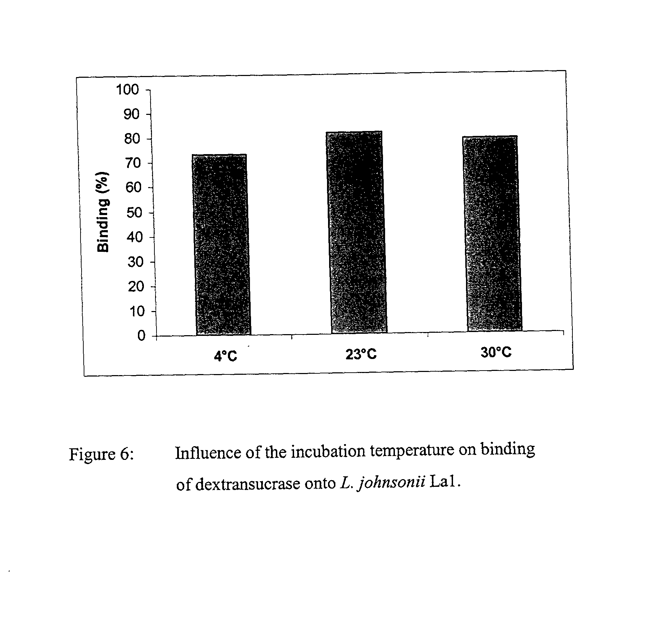

To analyze the binding capacity of dextransucrase at different temperatures, the buffers were equilibrated at 4°, 23°, 30° and 37°C (The binding parameters were an OD of 8.0 per ml, an enzyme concentration of 0.95 U/ml and incubation for 10 min at different temperatures). It was previously observed that dextransucrase is irreversibly inactivated at 37°C in solution. As shown in Fig. 6, the temperature at which the binding assay is performed had no effect on the capacity of dextransucrase to bind onto La1.

### Example 6

### Binding capacity of L. johnsonii La1 during growth

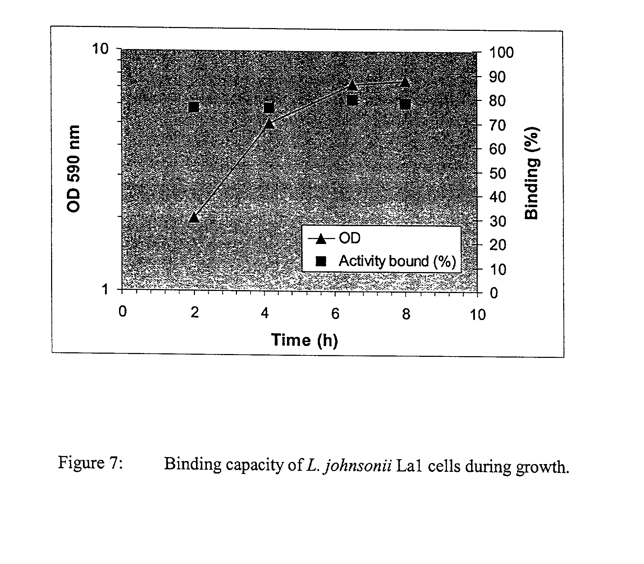

Since the cell surface is not constant but changes during growth of the bacteria, it was speculated that the binding efficiency might be influenced by the growth phase. Therefore *L*. *johnsonii* La1 was inoculated and samples were taken during growth to analyze the binding efficiency. Aliquots of 1 ml of an OD of 2.0 were used for the binding assay. The binding parameters were an OD of 4/ml, an enzyme concentration of 0.95 U/ml and incubation for 10 min on ice. Fig. 7 shows that there was no difference in binding efficiency during growth.

### Example 7

### Influence of dextran or dextran synthesis

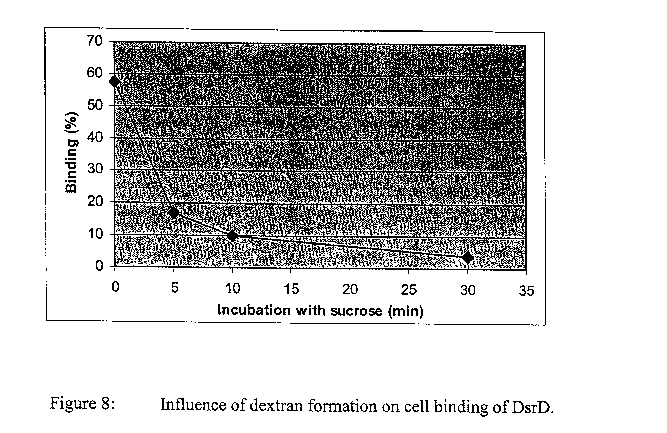

To analyze the influence of dextran on the enzymatic activity the dextransucrase preparation was preincubated at 30°C with sucrose at pH 5.2 to initiate dextran production. At different times the enzyme was diluted in binding buffer (to one fourth) and used for the binding assay. The dilution was performed to reduce the concentration of sucrose and the formation of dextran during the incubation with the La1 cells. The results of this experiment are shown in Fig. 8. It is clearly shown that the percentage of dextransucrase attached to the cells decreased rapidly after incubation with sucrose. Already after 5 min of incubation, less than 20% of dextransucrase remains attached to the cells.

From the experimental set-up it was not clear whether the dextran itself or rather the formation of the polymer mediates the inhibition of cell binding. Therefore a binding assay was performed in which increasing concentrations of purified Lcc4 dextran (0.2, 0.5, 1.5 and 2.7 mg/ml, final concentration of Lcc4 dextran, free of sucrose and other sugars) were present during the incubation with the cells. It could be shown, that the added dextran as such had no effect on cell attachment of dextransucrase. The previous experiments had shown that the formation of dextran in the presence of sucrose rather than the dextran itself inhibited binding of dextransucrase to cells.
In order to determine whether the dextransucrase previously attached to the cells would detach from the surface in the presence of sucrose, dextransucrase bound to La1 was incubated for different times in the buffer containing sucrose. At different time intervals, the mixture was centrifuged and the pellet was resuspended in 100 µl binding buffer. The remaining enzyme activity still bound to the cells was measured using Nelson-Somogyi assay.

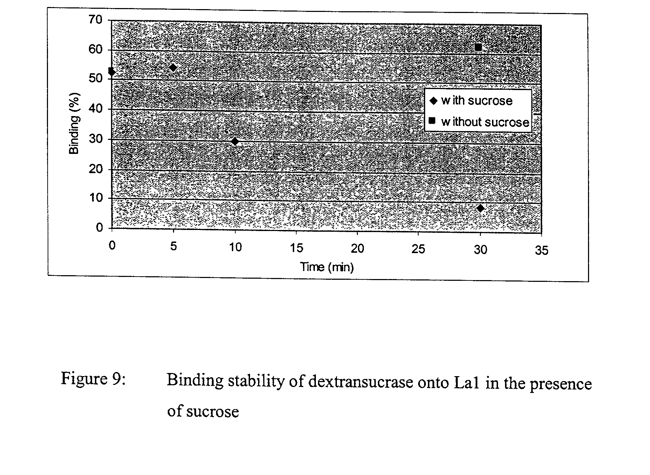

As may be seen from Fig. 9 within a few minutes cell-bound dextransucrase started to detach from the La1 surface in the presence of sucrose indicating that addition of sucrose may be used for starting the enzymatic activity of cell bound dextransucrase.

### Example 8

### Binding of increasing concentrations of dextransucrase onto La1

To analyze the maximum binding capacity of the cells, increasing concentrations of dextransucrase (between 0 and 0.78 U/ml) were added to an increasing quantity of *L. johnsonii* La1 cells (OD between 0 and 2.0). After 10 min incubation at 4°C, the dextransucrase activities of the cell pellet were determined. The used OD concentrations were as follows: 0, 0.5, 1, 1.5, and 2.0. For each OD, four different concentrations of dextransucrase were tested: 0, 0.24, 0.47, 0.78 U/ml.

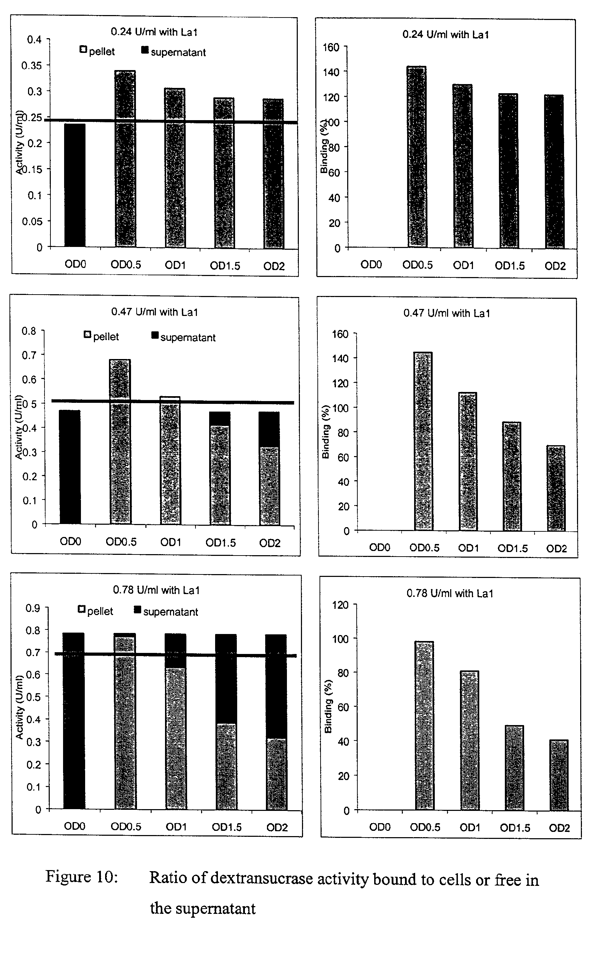

As shown in Fig 10, for a final enzyme activity of 0.24 U/ml, binding was about 100% for OD= 0.5 to 2.0. In any case, the number of binding sites would not be limited for the attachment of 0.24 U/ml. Moreover, it was shown that already with an OD of 0.5, the binding sites are numerous enough to link the 0.24 U/ml of dextransucrase. For an enzyme activity of 0.47, binding decreased below 80% for an OD of 2.0, and for an enzyme activity of 0.78 U/ml, binding decreased for whole range of OD. Therefore, binding of dextransucrase to La1 is almost complete at low dextransucrase activity independent of the OD. However at higher dextransucrase activity, binding is less efficient and decreases with OD and for a given OD, the cell-binding efficiency decreases with increasing the enzyme concentration.

### Binding of increasing concentrations of dextransucrase onto L. mesenteroides Lcc4

To study the binding of dextransucrase to Lcc4, it was necessary to minimize production of dextransucrase by the cells itself, which could result in a strong background in the binding assay. Therefore, Lcc4 was cultivated without the inducer sucrose in presence of glucose (medium Lcc4-glu) in a batch-culture. Biomass was harvested and binding assay was performed. OD concentrations were varied from 0 to 2.0 and dextransucrase was used in the range of from 0 to 0.79 U/ml. For each OD, four concentrations of dextransucrase were tested. The background activity of the cells without external addition of dextransucrase was measured and was found negligible.

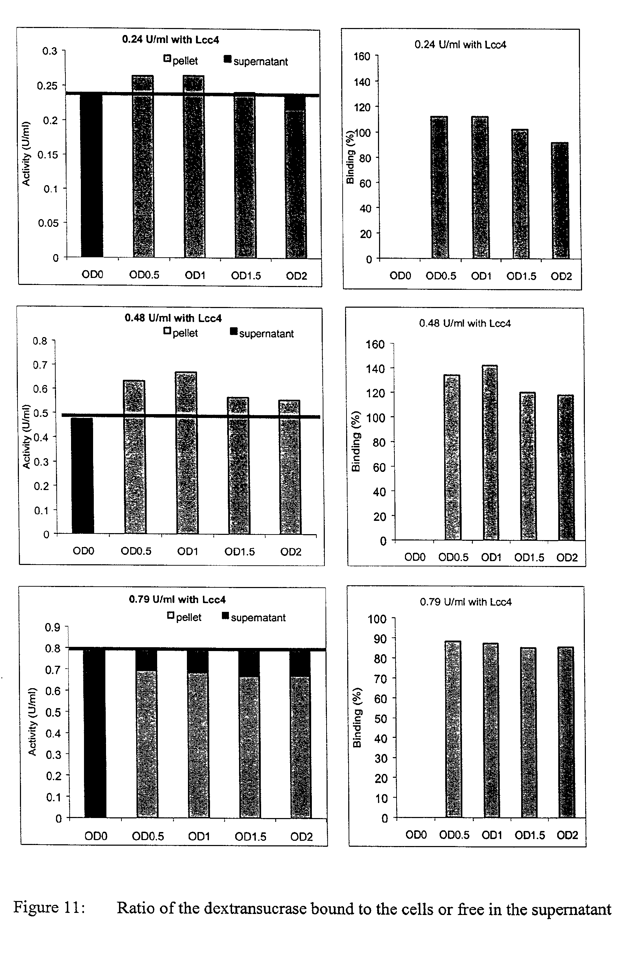

Fig. 11 shows that the binding of dextransucrase onto *L. mesenteroides* Lcc4 reached values higher than 80 % for all the experiments. In contrast to the binding to La1 (supra) and for the same conditions, the binding did not decrease with higher dextransucrase concentration or higher cell concentration. The binding always occurred in the optimum environment without any limitation. This indicates that even under the most unfavorable conditions when a maximum enzyme activity (0.79 U/ml) was used with the minimal quantity of cells (OD 0.5), still all enzyme could be attached. As described before for La1, calculations were performed on the enzyme activity bound to the cells and the enzyme activity removed in the supernatant.

### Example 9

### Determination of potential binding sites

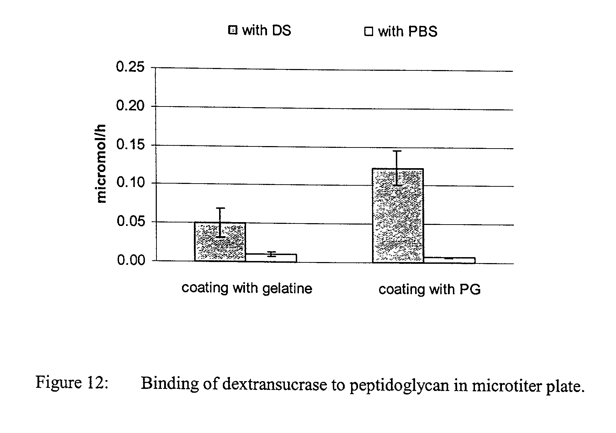

In order to determine potential binding sites on bacteria, plates were coated with peptidoglycan of *S. aureus* or gelatine for the control. Culture medium containing dextransucrase was added. After an incubation period, the plates were washed and sucrose buffer was added. The reducing sugar concentration was determined after incubation at 30°C with Nelson-Somogyi sucrose buffer at pH 5.2. For control the plates wells were incubated with PBS in parallel. Sucrose conversion is expressed in µmol/h. The results are shown in Fig. 12 indicating that peptidoglycan seems to be a binding site on the bacterial cell wall.

### Example 10

### Dextran synthesis during yogurt manufacturing

Yogurt samples A-F were produced according to the following protocol. A non-ropy *Streptococcus thermophilus* strain was precultured at 40°C on MSK inoculated with 1% stock culture until the pH reached 5.2 ± 0.05. *Lactobacillus johnsonii* La1 (NCC 533) was precultured on an appropriate medium (e.g. MRS) inoculated with 1% until the pH reached 3.95±0.05. Fermented medium (150 ml) was centrifuged to collect pellets of *Lactobacillus*. A pH-controlled fed-batch was performed with *Leuconostoc mesenteroides* (CNCM I-1692) on sucrose at a pH of 6.7. Final dextransucrase activity in the supernatant was 2 U/ml. Fermentation broth (400 ml) was centrifuged to remove cells, and supernatant was mixed with *Lactobacillus* cells to subsequently bind dextransucrase to the *Lactobacillus* cells. After incubation for 15 min at 4°C, incubation mixture was centrifuged to recover those *Lactobacillus* cells with dextransucrase bound to cells. Pellet was resuspended in 600 ml pasteurized skim milk and pH was adjusted to 5.2 with lactic acid or alternatively with supernatant of the *Lactobacillus* culture. A concentrated sucrose solution was added to final concentration of 64 g/l for sample A, 48.5 g/l for sample B, 34 g/l for sample C, 17.5 g/l for sample D, and 0 g/l for sample E. This reaction mixture was incubated overnight at 4°C to synthesize dextrans. 3 liters of pasteurized skim milk was preheated to 40°C and inoculated with 0.5% *S. thermophilus* preculture and 990 ml reaction mixture used for dextran synthesis. Fermentation was performed at 40°C. Desired final pH was 4.6±0.05 for a set yogurt and 4.4±0.05 for a stirred yogurt. For stirred yogurts, the fermented milk was cooled to 30°C and gently stirred at the end of the fermentation. Yogurt or fermented milk was rapidly cooled to 4°C to stop fermentation and stored at 4°C for at least 28 days to monitor organoleptic properties and determine viable cell counts. Sample F was produced by fermenting milk with a ropy *S. thermophilus* strain and *L. johnsonii* La1. Neither dextransucrase nor sucrose was added to this fermentation.

### Viscosity of products

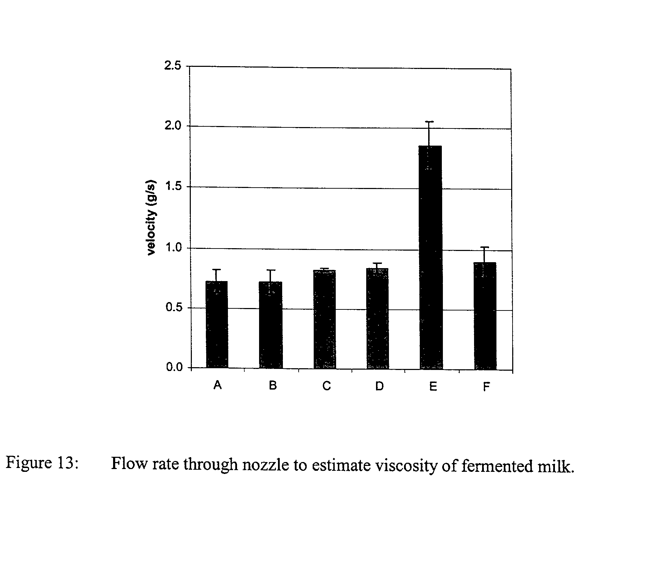

The viscosity of the stirred yogurts A-F was qualitatively assessed by measuring the flow rate through a nozzle of 3-mmol inner diameter for sample volumes of 15-40 ml. A high flow rate indicates a low viscosity. Results are reported on Figure 13 for samples A-F. As compared to sample E (no dextran synthesis), the viscosity of samples A-D is much higher. No clear difference could be detected among samples A-D. For the range of sucrose concentration used, the viscosity obtained was higher than for the ropy strain.

### Sensory analysis

Samples were evaluated 11 days after fermentation (storage at 4°C). Residual sugar analysis indicated that no sucrose was present in samples C-F.

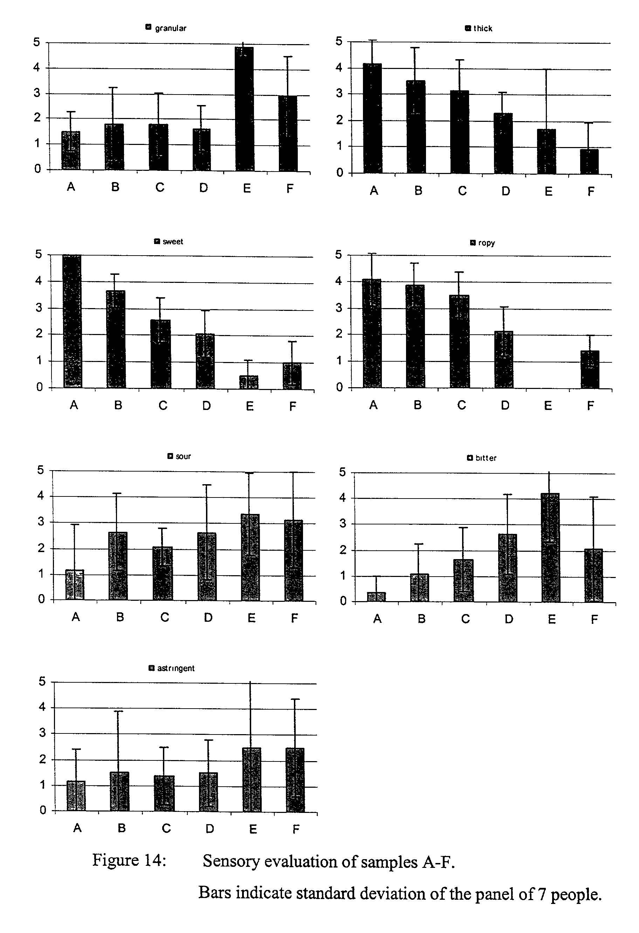

A panel consisting of 7 people has tested fermented milk. Attributes were: granular, thick, sweet, ropy, sour, bitter, astringent. Testers were asked to rank the samples for each attribute, the highest score correspond to the sample with the most pronounced character. Results are presented in Figure 14. Samples A-C were found equally ropy, sample D was slightly less ropy whereas sample E was found to be least ropy. By contrast sample E was the most granular. The panel found a regular decrease in thickness from sample A to F. Samples A-D were found sweet, although all added sucrose was converted to dextrans in samples C and D. Acidity and astringency do not seem to be affected by presence of dextrans. However, a lower bitterness is perceived when higher amounts of sucrose were used to synthesize dextrans (samples A-C). In general sample A and B were most preferred samples by the panel and no off-flavor was detected. *In-situ* synthesis of dextrans gave a pleasant mouthfeeling both for set and stirred yogurts, and both when ropy and non-ropy *S. thermophilus* strains were used.

## Claims

1. A method of preparing a fermented food product, which comprises the step of contacting the microorganism to be used for the fermentation process with a dextransucrase under conditions to enable a binding thereof to the cell wall of the microorganism.

2. The method according to claim 1, wherein the amount of dextransucrase bound to the microorganisms ranges from about 0.001-0.5 U/10⁶ cfu.

3. The method according to any of the preceding claims, wherein the microorganism is selected from the group consisting of *Lactobacillus, Bifidobacterium, Streptococcus, Leuconostoc, Pediococcus, Enterococcus*.

4. The method according to claim 3, wherein the microorganism is preferably *Lactobacillus johnsonii* (CNCM I-1225, CNCM I-2369 and CNCM I-2474), *Lactobacillus paracasei* (CNCM I-2116), *Lactobacillus acidophilus* (CNCM I-2332), *Lactobacillus reuteri, Lactobacillus animalis, Lactobacillus ruminis, Lactobacillus casei, Lactobacillus rhamnosus, Lactobacillus fermentum, Lactobacillus bulgaricus, Lactobacillus plantarum, Bifidobacterium bifidum* (CNCM I-2333), *Bifidobacterium infantis* (CNCM I-2334), *Bifidobacterium adolescentis* (CNCM I-2168), *Bifidobacterium longum, Bifidobacterium lactis* (ATCC 27536), *Bifidobacterium bifidum, Bifidobacterium animalis, Streptococcus thermophilus.*

5. The method according to any of the preceding claims, wherein the product to be prepared is selected from yoghurt, curd, cheese, fermented milks, milk based fermented products, ice cream, fermented cereal based products, milk based powders, water-based jellies, infant formula or pet food.

6. Use of a cell bound dextransucrase for the preparation of a food material.

7. The use according to claim 6, wherein the amount of dextransucrase bound to the cell surface of a microorganism is in the range of from 0.001-0.5 U/10⁶ cfu.

8. The use according to any of the claims 6 and 7, wherein the microorganism, to which a dextransucrase is bound is selected from the group consisting of *Lactobacillus, Bifidobacterium, Streptococcus, Leuconostoc, Pediococcus, Enterococcus.*

9. The use according to claim 8, wherein the microorganisms are preferably selected from *Lactobacillus johnsonii* (CNCM I-1225, CNCM I-2369 and CNCM I-2474), *Lactobacillus paracasei* (CNCM I-2116), *Lactobacillus acidophilus* (CNCM I-2332), *Bifidobacterium bifidum* (CNCM I-2333), *Bifidobacterium infantis* (CNCM I-2334) or *Bifidobacterium adolescentis* (CNCM I-2168).

10. The use according to any of the claims 6 to 9, wherein the food product is a yoghurt, curd, cheese, fermented milks, milk based fermented products, ice cream, fermented cereal based products, milk based powders, infant formula or pet food.

11. Bacterial concentrate having a dextransucrase bound to its surface in an amount of from 0.001-0.5 U/10⁶ cfu.

12. Use of a dextransucrase or a part thereof binding to a bacterial cell wall for transferring biological active substances into a food material.
